**Europäisches Patentamt**

**European Patent Office**

**Office européen des brevets**

(19)

(11) Veröffentlichungsnummer : **0 367 779 B1**

(12) **EUROPÄISCHE PATENTSCHRIFT**

(45) Veröffentlichungstag der Patentschrift :
**15.04.92 Patentblatt 92/16**

(51) Int. Cl.$^5$ : **A61F 2/36**

(21) Anmeldenummer : **88905696.6**

(22) Anmeldetag : **08.07.88**

(86) Internationale Anmeldenummer :
**PCT/DE88/00428**

(87) Internationale Veröffentlichungsnummer :
**WO 89/00414 26.01.89 Gazette 89/03**

(54) **ENDOPROTHESE FÜR EIN FEMURHÜFTGELENK.**

(30) Priorität : **10.07.87 DE 3722853**

(43) Veröffentlichungstag der Anmeldung :
**16.05.90 Patentblatt 90/20**

(45) Bekanntmachung des Hinweises auf die
Patenterteilung :
**15.04.92 Patentblatt 92/16**

(84) Benannte Vertragsstaaten :
**AT BE CH FR GB IT LI NL SE**

(56) Entgegenhaltungen :
**EP-A- 0 207 299**
**WO-A-86/00011**
**DE-A- 3 304 476**
**FR-A- 2 483 218**

(73) Patentinhaber :
**KERNFORSCHUNGSZENTRUM KARLSRUHE
GMBH**
**Weberstrasse 5 Postfach 3640**
**W-7500 Karlsruhe 1 (DE)**

(72) Erfinder : **MATTHECK, Claus**
**Siemensstrasse 6**
**W-6729 Leimersheim (DE)**
Erfinder : **BÖRNER, Martin**
**Höchster Str. 14 d**
**W-6231 Schwalbach (DE)**

**Beschreibung**

Endoprothese für ein Femurhüftgelenk, deren Prothesenschaft mit einem auf einer Knochenresektionsfläche abstützbaren Prothesenkragen mit einem die aufsetzbare Gelenkkugel tragenden Klemmkonus versehen ist, wobei die Endoprothese mindestens eine als Zuggurt an ihr angreifende Schraube aufweist, welche lateral schräg nach unten und außen ausgerichtet ist und in einer entsprechend verlaufenden Bohrung des Femurknochens plazierbar ist.

Eine derartige Zuggurtungs-Endoprothese ist aus der DE-OS 35 22 692 A1 bekannt. Die Zugschraube des Prothesenschaftes wird jedoch bereits in der Veröffentlichung "Die Zuggurtung-Hüftendoprothese", Arch. orthop. Unfall-Chir. 86, 1 - 14 (1976) beschrieben. Wie hieraus zu entnehmen ist, wird ein relativ kurzer Schaft in den Femurknochen getrieben (Seite 11, 7. Absatz und Abb. 6, Seite 9), da dem Schaft nur noch eine untergeordnete Bedeutung zugemessen ist. Der Schaft mit breiter, medialer Auflagefläche und einer Längsnut dient nur noch der Übertragung relativ geringer Kräfte.

Der bei Schaftprothesen auftretende Kippeffekt darf jedoch aus medizinisch-technischen Gründen nicht unberücksichtigt bleiben. Bei Dauerbelastung übt die dicke unelastische Schaftspitze gegen die relativ weiche Knocheninnenwand direkt einen Druck aus. Auch muß der Kraftfluß aus dem unteren Ende des Prothesenschaftes in den Knochen umgelenkt werden, was dort im Knochen lokal hohe Kerbspannungen induziert. Dieser führt zuerst zu einer Verengung der Markhöhle durch die Verfestigung der Spongiosa und zu einer Verdickung der Knochenwandung. Daraufhin setzt sich die Prothesenschaftspitze auf diesem Knochenstöpsel in der Markhöhle auf, so daß der Kraftfluß fast nur noch über die Prothese in den Stöpsel gelenkt wird. Eine Zurückbildung der Knochenwandung über dem Stöpsel und schlimmstenfalls Lockerungen sowie Prothesen- und Knochenbruch können die Folge sein.

Die der Erfindung zugrundeliegende Aufgabe besteht darin, die e. g. Halterung derart auszubilden, daß der Prothesenschaft zementlos und ohne Steifigkeitssprünge im Femurknochen, auch unter höchsten Anforderungen, sitzen bleibt.

Die Lösung ist in den kennzeichnenden Merkmalen des Anspruches 1 beschrieben.

Die übrigen Ansprüche geben vorteilhafte Weiterbildungen und Ausführungsformen der Erfindung an.

Die besonderen Vorteile des erfindungsgemäßen Vorschlags bestehen darin, daß durch die zementlose Plazierung eines vergleichsweise biegeweichen Prothesenschaftes in einem ebenfalls vergleichsweise biegeweichen Marknagel, der bis unten in den Knochen reicht, die Ausbildung eines Knochenstöpsels und damit ein Kippeffekt von vornherein vermieden wird. Die Belastung wird verteilt über die gesamte Nagellänge eingeleitet. Um dieses biegeweiche System zusätzlich zu stabilisieren, ist auf der Zugseite, lateral außen, noch der Zuggurtungsbügel vorgesehen. Das wesentlich Neue ist somit die Kombination von Prothesenschaft und Marknagel.

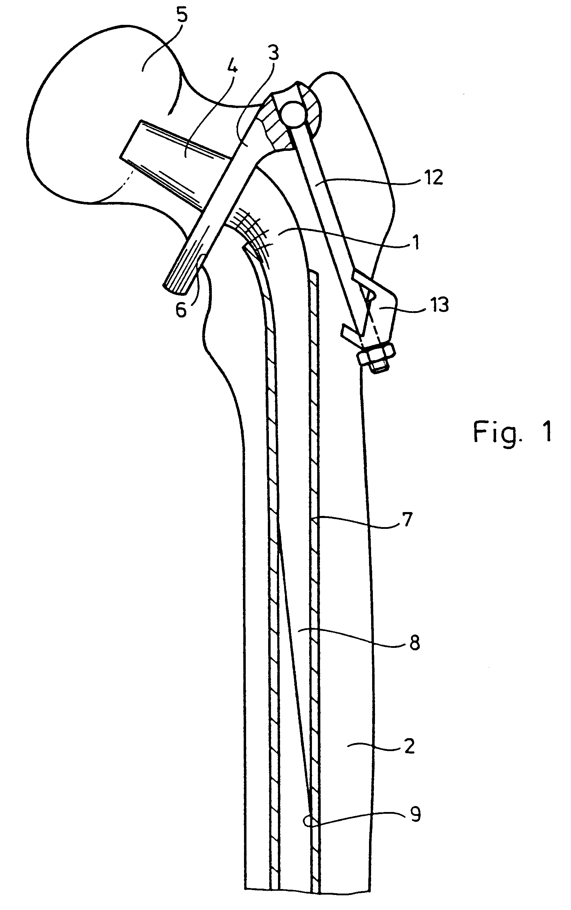

Die Erfindung wird im folgenden anhand von drei Ausführungsbeispielen mittels der Fig. 1 - 3 näher beschrieben.

Für alle drei Ausführungsbeispiele (Fig. 1 - 3) gilt, daß die Femurhüftendoprothese einen gekrümmten Schaft 1 besitzt, der im Röhrenknochen 2 gehaltert werden soll. Er setzt sich fort mit einem Prothesenkragen 3 und einem Klemmkonus 4 zur Aufnahme einer Gelenkkugel 5. Der Prothesenkragen 3 sitzt auf einer flächenförmigen Resektionsfläche 6 auf.

Vor dem Einsetzen des Profhesenschaftes 1 wird erfindungsgemäß in den Femurknochen 2 ein Marknagel 7 eingeführt. Er ist hohl ausgebildet, vorzugsweise bis an sein Ende geschlitzt und reicht über die gesamte Länge des Femurknochens 2 bzw. der Markhöhle hinweg. Die Querschniffsdimensionen des Marknagels 7 sind, zumindest an ihrem oberen Ende, den Abmessungen des einzusetzenden Schaftoberteils angepaßt. Das freie Schaftende 8 ist jedoch biegeweich ausgebildet. Das Innenprofil des Nagels ist vorteilhaft kreisförmig.

Gemäß der Ausführung nach Fig. 1 läuft das Schaftende 8 zum gelenkfernen Bereich spitz aus (verjüngt sich), wobei es an der lateralen Innenfläche 9 des Marknagels 7 zur Anlage kommt. Als Material kommt z. B. Implantatstahl in Betracht, aber auch Faserverbunde sind geeignet.

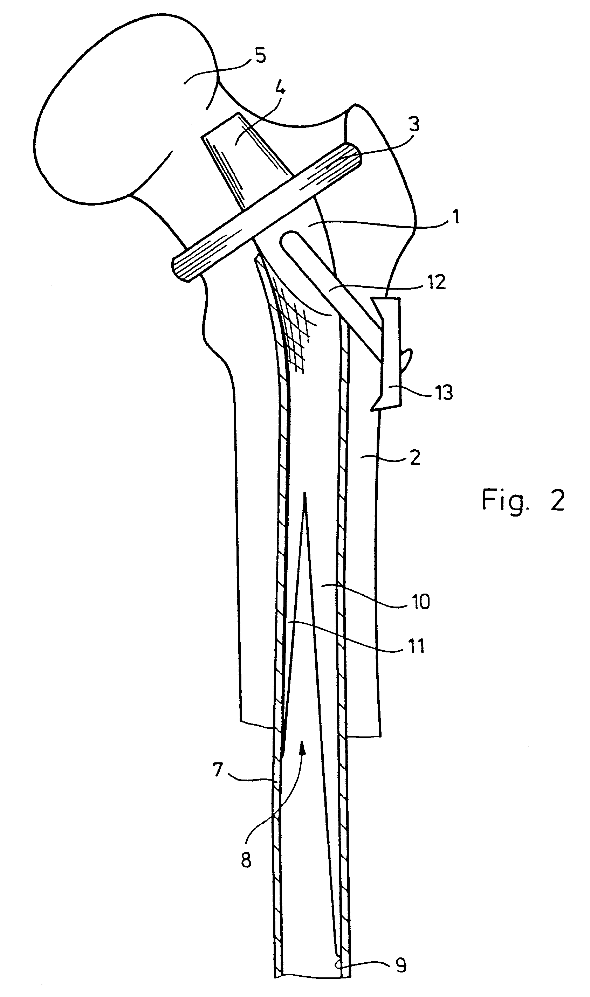

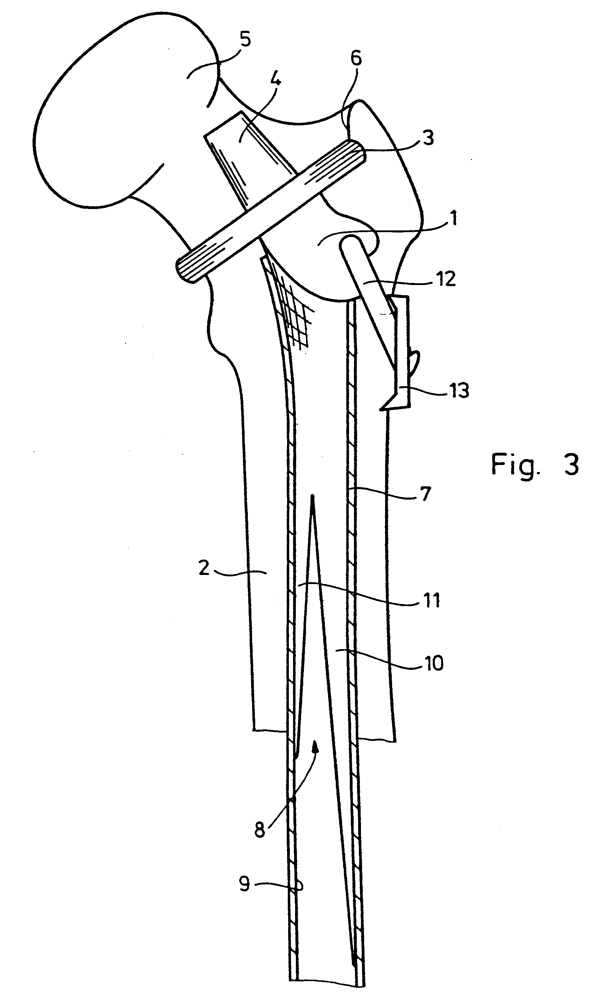

Gemäß den Ausführungen nach Fig. 2 und 3 ist das Schaftende 8 gegabelt ausgeführt. Jeweils zwei zur Spitze hin sich verjüngende Zinken 10, 11 sind vorgesehen, wobei sich der etwas kürzere (11) an der Innenwandung 9 medial und der längere (10) an der Innenwand 9 lateral anlegt. Durch diese Gabelung wird die Elastizität des Schaftendes 8 noch erhöht und die Belastung verteilt über die gesamte Marknagellänge auf den Femurknochen 2 übertragen.

Bei allen drei Ausführungsformen ist zusätzlich eine Zuggurt-Verankerung vorgesehen. Hierzu dient mindestens eine Schraube 12 (Kortikalisschraube oder in einer lateralen Auflageplatte fixierten gewöhnlichen Schraube mit Metallgewinde) bzw. Bügel mit Zackenscheibe 13, die oder der im Femurknochen 2 verläuft und entweder am Prothesenkragen 3 (Fig. 1) oder unterhalb des Prothesenkragens 3 am Schaft 1 in der Achse (Fig. 2) oder außerhalb der Achse (Fig. 3) befestigt sind. Weitere Befestigungselemente (Verriegelungsnägel) können für den Marknagel 7 vorgesehen sein.

**Patentansprüche**

1. Endoprothese für ein Femurhüftgelenk, deren Prothesenschaft (1) mit einem auf einer Knochenresektionsfläche abstützbaren Prothesenkragen (3) mit einem die aufsetzbare Gelenkkugel (5) tragenden Klemmkonus (4) versehen ist, wobei die Endoprothese mindestens eine als Zuggurt an ihr angreifende Schraube (12) aufweist, welche lateral schräg nach unten und außen gerichtet ist und in einer entsprechend verlaufenden Bohrung des Femurknochens plazierbar ist, dadurch gekennzeichnet, daß der Prothesenschaft (1) in einem in die Markhöhle eintreibbaren hohlen Marknagel (7) untergebracht ist, welcher Bestandteil der Endoprothese ist und dessen Querschnittsdimensionen den Abemessungen des Oberteils des einzusetzenden Prothesenschafts (1) angepaßt ist, und daß der Prothesenschaft (1) im distalen Bereich (8) derart elastisch ausgebildet ist, daß die Belastung über die gesamte Länge des Marknagels (7) verteilt auf den Femurknochen (2) übertragbar ist, wobei der Prothesenschaft (1) sich zum gelenkfernen Bereich (8) hin verjüngt und lateral von innen an der Wandung (9) des Marknagels (7) anliegt oder wobei der Prothesenschaft (1) als Gabel mit zwei sich zur Spitze (8) hin verjüngenden, lateral und medial an der Innenwandung (9) des Marknagels (7) anliegenden Zinken (10, 11) ausgebildet ist.

2. Prothese nach Anspruch 1, dadurch gekennzeichnet, daß die Schraube (12) entweder am Prothesenkragen (3) oder darunter am Prothesenschaft (1) angeordnet ist.

3. Prothese nach Anspruch 1 und 2, dadurch gekennzeichnet, daß der Marknagel (7) über seine gesamte Länge geschlitzt ausgebildet ist.

**Claims**

1. Endoprosthesis for a femural hip joint, the shaft (1) of which prosthesis is provided with a prosthesis collar (3), which is supportable on a resected bone surface and has a blocking cone (4), which bears the mountable ball head (5), the endoprosthesis having at least one screw (12), which grips the prosthesis as a tensioning stay and extends obliquely downwardly and outwardly and is insertable in a correspondingly extending lateral bore in the femur, characterised in that the prosthesis shaft (1) is accommodated in a hollow medullary nail, which is drivable into the medullary canal and is a component part of the endoprosthesis, and the cross-sectional dimensions of said nail are adapted to the dimensions of the upper portion of the prosthesis shaft (1) to be inserted, and in that the prosthesis shaft (1) is resilient in the distal region (8) in such a manner that the load is distributed over the entire length of the medullary nail (7) and is transferable to the femur (2), the prosthesis shaft (1) tapering towards the region (8) remote from the joint and abutting against the lateral wall (9) of the medullary nail (7) laterally from internally, or the prosthesis shaft (1) is a bifurcated member having two prongs (10, 11), which taper towards the point (8) and abut laterally and medially against the inner wall (9) of the medullary nail (7).

2. Prosthesis according to claim 1, characterised in that the screw (12) is disposed either on the prosthesis collar (3) or therebeneath on the prosthesis shaft (1).

3. Prosthesis according to claims 1 and 2, characterised in that the medullary nail (7) is slotted over its entire length.

**Revendications**

1. Endoprothèse pour une articulation coxofémorale, dont la tige de prothèse (1) est munie d'un collet de prothèse (3) pouvant s'appuyer sur une surface de section d'os avec un cône de serrage (4) supportant l'articulation sphérique applicable (5), l'endoprothèse comprenant au moins une vis (12) appliquée à elle comme membrure travaillant à l'extension, qui est dirigée latéralement inclinée vers le bas et vers l'extérieur et qui peut être mise en place dans un alésage préparé en conséquence dans le fémur, endoprothèse caractérisée en ce que la tige de prothèse (1) est introduite dans une broche médullaire (7) creuse pouvant être introduite dans le canal médullaire, broche qui est un composant de l'endoprothèse et dont les dimensions de section transversale sont adaptées aux dimensions de la partie supérieure de la tige de prothèse (1) à mettre en place, et en ce que la tige de prothèse (1) est réalisée souple dans la zone distale (8) de sorte, que la charge peut être supportée sur le fémur (2) par répartition sur toute la longueur de la broche médullaire (7), la tige de prothèse (1) s'amincissant en allant vers la zone (8) éloignée de l'articulation et s'appuyant latéralement de l'intérieur

3

sur la paroi (9) de la broche médullaire (7) ou bien la tige de prothèse (1) étant réalisée en fourche avec deux dents (10, 11) s'amincissant en allant vers la pointe (8) en s'appuyant latéralement et médialement sur la paroi interne (9) de la broche médullaire (7).

2. Prothèse selon la revendication 1, caractérisée en ce que la vis (12) est placée soit sur le collet de prothèse (3), soit en dessous sur la tige de prothèse (1).

3. Prothèse selon les revendications 1 et 2, caractérisée en ce que la broche médullaire (7) est réalisée fendue sur toute sa longueur.

Fig. 1

Fig. 2

Fig. 3